# EUROPÄISCHE PATENTSCHRIFT

(11) **EP 1 135 998 B1**
(45) Veröffentlichungstag und Bekanntmachung des Hinweises auf die Patenterteilung: **13.08.2003**
(21) Anmeldenummer: 01114124.9
(22) Anmeldetag: 21.04.1998
(51) Int. Cl.: A23L 3/3517

(54) **Verwendung von synthetischen Triglyceriden auf Basis konjugierter Linolsäure als Antioxidans in Lebensmittel**
Use of synthetic triglycerides based on conjugated linoleic acid as antioxydant in foodstuffs
Utilisation de glycérides synthétiques à base d'acide linoléique conjugué comme antioxydant dans les produits alimentaires

(30) Priorität: 30.04.1997 DE 19718245
(43) Veröffentlichungstag der Anmeldung: 26.09.2001
(62) Teilanmeldung aus: 98921473.9
(73) Patentinhaber: Cognis Deutschland GmbH & Co. KG, 40589 Düsseldorf (DE)
(72) Erfinder: Timmermann, Franz, 89257 Illertissen (DE); Gaupp, Rolf, 89165 Dietenheim (DE); Gierke, Jürgen, 89257 Illertissen (DE); von Kries, Rainer, 89257 Illertissen (DE); Adams, Wolfgang, 68074 Meckenbeuren (DE); Sander, Andreas, 89257 Illertissen (DE)

(56) Entgegenhaltungen:
- WO-A-96/34846
- US-A- 5 017 614
- PATENT ABSTRACTS OF JAPAN vol. 1995, no. 01, 28. Februar 1995 (1995-02-28) & JP 06 276939 A (SNOW BRAND MILK PROD CO LTD), 4. Oktober 1994 (1994-10-04)

## Beschreibung

### Gebiet der Erfindung

Die Erfindung betrifft die Verwendung von synthetischen Triglyceriden auf Basis konjugierter Linolsäure als Antioxidans in Lebensmitteln.

### Stand der Technik

Die mehrfach ungesättigten ω-3 und ω-6 Fettsäuren wie α-Linolensäure, Linolsäure zählen zu den für die Säugetiere und den Menschen essentiellen Fettsäuren. Neben der Linolsäure existieren in der Natur noch andere isomere Octadecadiensäuren. Diese zeichnen sich durch konjugierte Doppelbindungen an den C-Atomen 9 und 11, 10 und 12 sowie 11 und 13 aus. Diese isomeren Octadecadiensäuren werden in der wissenschaftlichen Literatur unter dem Begriff konjugierte Linolsäuren (Abkürzung: CLA) zusammengefasst und haben in letzter Zeit zunehmend Beachtung gefunden [vgl. **NUTRITION, 19(6) 1995**]. Konjugierte Linolsäuren finden sich als Bestandteile in verschiedenen Lebensmitteln. Ihre Hauptquelle sind die tierischen Lebensmittel, aber auch in Milch und Milchprodukten sind bedeutende CLA-Mengen enthalten. Des weiteren wurde in verschiedenen Ölen und Fetten CLA nachgewiesen, wobei die Konzentration in pflanzlichen Ölen bedeutend niedriger war als jene in tierischen Fetten (vgl. **J.Food Compos. AnaL 5, 185-197 (1992)]**

Über die Bedeutung der CLA auf den Organismus haben verschiedenen Arbeitsgruppen berichtet neuerdings wurde von Shultz et al. über die hemmende Wirkung auf das in vitro-Wachstum von menschlichen Krebszellen berichtet **[Carcinogenesis 8, 1881-1887 (1987); Cancer Lett. 63,125-133 (1992)].** In in vitro-Versuchen wurde die Wirkung der CLA auf das Wachstum von menschlichen bösartigen Melanomen, Dickdarm- und Brustkrebszellen überprüft. In den Kulturmedien zeigte sich eine signifikante Reduktion im Wachstum der Krebszellen, die mit CLA behandelt wurden im Vergleich zu Kontrollkulturen. Der Mechanismus, über welchen CLA eine antikarzinogene Aktivität ausübt, ist unbekannt Daneben weist CLA eine hohe antioxidative Wirkung auf, womit beispielsweise die Lipidperoxidation gehemmt werden kann **[Atherosclerosis 108, 19-25 (1994)].** Weiterhin wurde beispielsweise der Zusatz von konjugierter Linolsäure zu Nahrungsmitteln zum Zweck der Farbstabilisierung untersucht **[JP 06/276939 A2].** Der Einsatz von konjugierter Linolsäure in der Tierfütterung und in diesem Zusammenhang auch in der menschlichen Ernährung ist z. B. aus der **WO 96/06605** bekannt. Diese Anmeldung behandelt die Reduktion des Körperfettgehalts in der Tierernährung im Rahmen der Darstellung der Aufgabe wird auch die Übertragung auf den Menschen angesprochen. Insbesondere die Verwendung einer Fettemulsion enthaltend 0,5 bis 2 Gew.-% konjugierter Linolsäure zur oralen oder intravenösen Ernährung beim Menschen wird genannt. Aus der **EP 0579901 B1** ist der Einsatz von konjugierter Linolsäure zur Vermeidung eines Gewichtsverlustes bzw. einer Verringerung der Gewichtszunahme oder von Annorexia die durch Immunstimulation verursacht wurde bei Menschen oder Tieren bekannt. Die **WO 94/16690** beschäftigt sich mit der Effizienzverbesserung der Nahrungsverwertung bei Tieren, indem eine wirksame Menge von konjugierter Linolsäure verabreicht wird.

Im Zusammenhang mit den zahlreichen positiven Effekte von konjugierter Linolsäure, wie sie in intensiven Studien insbesondere an Tieren und Gewebekulturen nachgewiesen wurden, wurde auch der Einsatz in Nahrungsmitteln für den Menschen diskutiert. Die Anwendung von freier konjugierter Linolsäure in Nahrungsmitteln und Pharmaka wird jedoch dadurch begrenzt, dass es zum einen bei der Einarbeitung in komplexe Nahrungsmittel zu unerwünschten Reaktionen mit anderen Nahrungsmittelbestandteilen kommen kann, zum anderen aber auch durch den unangenehmen Geschmack und Geruch von konjugierter Linolsäure die zur Ablehnung beim Verbraucher führen können. Ein weiterer Nachteil ergibt sich daraus, dass freie Fettsäuren unter die Lebensmittelzusatzstoffregelung fallen und somit ihre Anwendung in Nahrungsmitteln eingeschränkt wird.

Die komplexe Aufgabe der vorliegenden Erfindung bestand somit darin ein Substitut für konjugierte Linolsäure für den antioxidativen Einsatz im Lebensmittelbereich zu finden.

### Beschreibung der Erfindung

Gegenstand der vorliegenden Erfindung ist die Verwendung synthetischer Triglyceride der Formel **(I),** 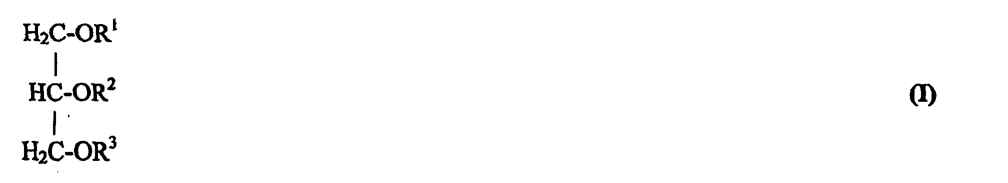 in der R¹, R² und R³ unabhängig voneinander für Fettsäurereste mit 6 bis 24 C-Atomen stehen, mit der Maßgabe, dass mindestens ein Rest R¹, R² oder R³ für einen konjugierten Linolsäurerest steht, als Antioxidans in Lebensmitteln

Überraschenderweise wurde gefunden, dass die erfindungsgemäßen Triglyceride bezüglich der antioxidativen und farbstabilisierenden Wirkung in Nahrungsmitteln vergleichbare Ergebnisse zeigen, wie die reine konjugierte Linolsäure.. Insbesondere durch ihre Lipophilie lassen sie sich auch in fetthaltige Produkte leicht einarbeiten und werden auch vom tierischen oder menschlichen Organismus gut resorbiert. Gleichzeitig zeichnen sie sich durch einen äußerst geringen Eigengeruch und Eigengeschmack aus. Sie sind daher bezüglich ihrer organoleptischen Eigenschaften der freien konjugierten Linolsäure weit überlegen. Dies ermöglicht beispielsweise eine höhere Dosierung der Triglyceride.

### Triglyceride

In den im Rahmen der vorliegenden Erfindung zu verwendenden Triglyceriden gemäß Formel (I) steht mindestens einer der Reste R¹, R² oder R³ für einen konjugierten Linolsäurerest, während die übrigen Reste für beliebige Fettsäurereste mit 6 bis 24 C-Atomen stehen. Besonders bevorzugt sind jedoch solche Triglyceride, die im statistischen Mittel mehr als 2 konjugierte Linolsäurereste pro Triglycerid aufweisen und insbesondere bevorzugt sind Triglyceride bei denen die Reste R¹, R² und R³ für einen konjugierten Linolsäurerest stehen. Im Rahmen der vorliegenden Erfindung sind unter Triglyceriden auch technische Mischungen von Mono-, Diund Triglyceriden zu verstehen, wie sie insbesondere bei der direkten Veresterung von Glycerin mit konjugierter Linolsäure anfallen. Eine typische Zusammensetzung wie sie besonders bevorzugt eingesetzt wird und insbesondere bei der Veresterung von Glycerin mit konjugierter Linolsäure erhalten wird, enthält 60 bis 98, vorzugsweise 80 bis 98 Gew.-% eines Triglycerids der konjugierten Linolsäure, 1 bis 40, vorzugsweise 1 bis 20 Gew.-% eines Diglycerids der konjugierten Linolsäure und maximal 2, vorzugsweise maximal 1 Gew.-% eines Monoglycerids der konjugierten Linolsäure. Gleichzeitig weist das erfindungsgemäß einzusetzende Glycerid eine Säurezahl von maximal 5, vorzugsweise maximal 3 auf, sowie eine Hydroxylzahl kleiner 40, vorzugsweise kleiner 30 und eine Peroxidzahl unterhalb von 4, vorzugsweise kleiner 2.

### Fettsäuren

Unter Fettsäuren im Sinne der vorliegenden Erfindung sind aliphatische Carbonsäuren der Formel **(II)** zu verstehen,

**R**^{**4**}**CO-OH (II)**

in der R⁴CO für einen aliphatischen, linearen oder verzweigten Acylrest mit 6 bis 24 Kohlenstoffatomen und 0 und/oder 1, 2 oder 3 Doppelbindungen steht. Typische Beispiele sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isosteansäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkern- oder Talgfettsäure. Unter konjugierter Linolsäure sind erfindungsgemäß vorzugsweise die Hauptisomere 9,11 Octadecadiensäure und 10,12 Octadecadiensäure zu verstehen sowie jedoch beliebige Isomerenmischungen, wie sie üblicherweise bei der Herstellung konjugierter Linolsäure anfallen.

### Umesterung

Die erfindungsgemäß als Ausgangsmaterialien einzusetzenden Fettsäureglyceride können die üblichen natürlichen pflanzlichen oder tierischen Fette oder Öle sein. Hierzu gehören beispielsweise Palmöl, Palmkernöl, Baumwollsaatöl, Rapsöl, Kokosöl, Erdnussöl, Olivenöl, Leinöl, Babassuöl, Teeöl, Olivenkernöl, Meadowfoamöl, Chaulmoograöl, Korianderöl, Sojaöl, Rizinusöl, Lardöl, Rindertalg, Schweineschmalz, Fischöl, sowie Sonnenblumenöl und Rapsöl der alten und neuen Züchtung. Die Hauptbestandteile dieser Fette und Öle sind Glyceride verschiedener Arten von Fettsäuren, die beträchtliche Mengen an Verunreinigungen wie etwa Aldehydverbindungen, Phospholipidverbindungen und freie Fettsäuren enthalten. Diese Materialien können direkt oder nach vorheriger Aufreinigung eingesetzt werden. In manchen Fällen ist es besonders empfehlenswert, die freien Fettsäuren in einer vorgeschalteten Reaktion mit niederen Alkoholen zu verestern. Diese Triglyceride werden gemäß den üblichen aus dem Stand der Technik bekannten Methoden mittels saurer und/ oder basischer Umesterung mit Fettsäuremischungen, die mindestens 50 insbesondere 70 bis 100 Gew.-% konjugierte Linolsäure enthalten unter Inertgas umgeestert. Als Inertgas wird vorzugsweise Stickstoff eingesetzt. Die Reaktion wird bevorzugt bei einer Temperatur im Bereich von 180 bis 240 °C durchgeführt, dabei ist in einer besonders bevorzugten Ausführungsform eine möglichst geringe Heizrate im Bereich von 0,2 bis 10, vorzugsweise 0,5 bis 3 K pro Minute einzuhalten. Als Katalysatoren eignen sich die üblichen für Ver- bzw. Umesterungen aus dem Stand der Technik bekannten. Es handelt sich dabei beispielsweise um Alkali- und/oder Erdalkalimetallalkoholate oder -hydroxide, insbesondere Natriummethanolat und /oder Natriumglycerat. Weiterhin bevorzugt ist der Einsatz von Acetaten, wie Zink- und/ oder Magnesiumacetat oder auch Titanaten und insbesondere von Zinnverbindungen, Organozinnverbindungen wie beispielsweise Dibutylzinndiacetat oder von Zinnsalzen.

### Veresterung

In einer besonders bevorzugten Ausführungsform erfolgt die Herstellung der erfindungsgemäßen Triglyceride statt über die Umesterung über die direkte Veresterung von Glycerin gemäß den üblichen aus dem Stand der Technik bekannten Methoden, mit einem Fettsäuregemisch, welches mindestens 50, vorzugsweise 70 bis 100 Gew.-% konjugierte Linolsäure enthält unter Inertgas, ebenfalls vorzugsweise Stickstoff. Insbesondere bevorzugt ist die Veresterung von Glycerin mit 100 Gew.-% konjugierter Linolsäure. Dabei erhält man üblicherweise technische Mischungen von Mono-, Di- und Triglyceriden der konjugierten Linolsäure. Erfindungsgemäß können sowohl diese Mischungen direkt eingesetzt werden, als auch nach weiterer Aufreinigung. Bezüglich Temperatur, Heizrate und Katalysator gilt das gleiche wie bereits im Rahmen der Umesterung aufgeführt. Im Anschluss an die Veresterung bzw. Umesterung gibt man vorzugsweise 0,01 bis 1 Gew.-% eines Antioxidans zu. In einer weiteren bevorzugten Ausführungsform reinigt man das erhaltene Triglycerid noch mit Hilfe eines Dünnschichtverdampfers auf und setzt danach erneut 0,01 bis 1 Gew.-% eines Antioxidans hinzu.

### Antioxidantien

Als Antioxidantien im Sinne der vorliegenden Erfindung eignen sich alle üblichen natürlichen Antioxidantien, wie sie insbesondere auch im pharmazeutischen Bereich und in der Ernährung zugelassen sind, hierunter fallen VitaminC und Vitamin C-derivate wie beispielsweise Ascorbylpalmitat, Carotinoide, Rosmarinextrakte und / oder synthetische Antioxidantien wie beispielsweise BHA, BHT, TBHQ oder Gallate und insbesondere verschiedene Vitamin E-Derivate, wie beispielsweise Coviox® T 70.

### Beispiele

Im folgenden wird die Herstellung der erfindungsgemäß einzusetzenden Triglyceride auf Basis konjugierter Linolsäure an Hand von zwei Beispielen illustriert.

### Beispiel 1

92,1 kg Glycerin und 841,5 kg konjugierte Linolsäure wurden unter Stickstoff vorgewärmt auf ca. 80°C in einem Reaktor geleitet und unter Rühren 0,62 kg des Zinnschliff hinzugefügt. Anschließend wurde auf 30 mbar evakuiert, weitergerührt für 10 Minuten mit Stickstoff belüftet. Die Erwärmung erfolgt unter Stickstoffspülung mit einer Heizrate von 1 K pro Minute innerhalb von einer Stunde auf 150°C dabei wurde der Druck auf 800 mbar abgesenkt. Innerhalb einer weiteren Stunde wurde auf 210°C erwärmt und bei dieser Temperatur für 2 Stunden gerührt. Anschließend wurde der Ansatz erneut innerhalb von 30 Minuten auf 30 mbar evakuiert und gerührt bis zum Erreichen einer Säurezahl von 15. Der Ansatz wurde im Vakuum auf 90°C abgekühlt und mit Stickstoff belüftet, anschließend wurde zur Ausfällung des Katalysators Phosphorsäure hinzugegeben, für 15 Minuten gerührt und nach Zugabe von Perlite über eine Filterpresse in einen mit Stickstoff gespülten Vorlagebehälter filtriert und 0,1 Gew.-% Coviox T-70 als Stabilisator hinzugeführt.

### Beispiel 2

Das Verfahren wurde wie im Beispiel 1 durchgeführt im Anschluss wurde das Rohprodukt jedoch in einem Dünnschichtverdampfer bei 230°C in Gegenwart von Strippdampf desodoriert. Dem Endprodukt wurden weitere 0,2 Gew.-% Coviox T-70 zur Stabilisierung zugegeben.

## Patentansprüche

1. Verwendung synthetischer Triglyceride der Formel **(I)** 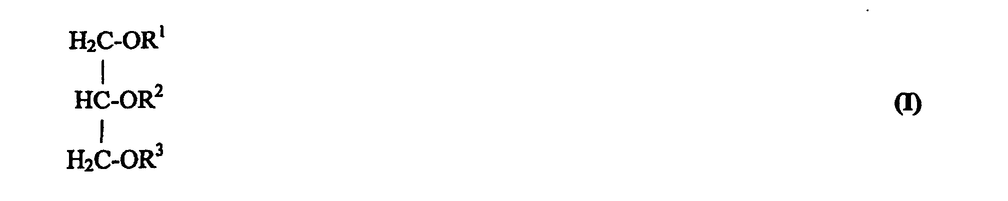 in der R¹, R² und R³ unabhängig voneinander für Fettsäurereste mit 6 bis 24 C-Atomen stehen, mit der Maßgabe, dass mindestens ein Rest R¹, R² oder R³ für einen konjugierten Linolsäurerest steht, als Antioxidans in Lebensmitteln

## Claims

1. The use of synthetic triglycerides corresponding to formula **(I):** 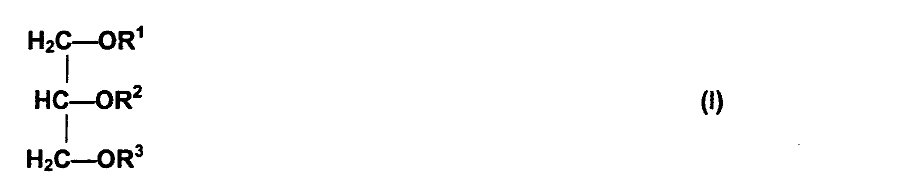 in which R¹, R² and R³ independently of one another represent C₆₋₂₄ fatty acid residues, with the proviso that at least one substituent R¹, R² or R³ is a conjugated linoleic acid residue,
as an antioxidant in foods.

## Revendications

1. Utilisation de triglycérides synthétiques de formule (I), 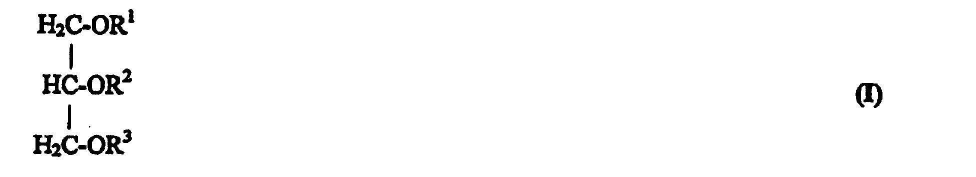 dans laquelle R¹, R² et R³ indépendamment les uns des autres, représentent des restes d'acide gras ayant de 6 à 24 atomes de carbone, avec la précision qu'au moins un reste R¹, R² ou R³ représente un reste d'acide linoléique conjugué, comme antioxydant dans les produits alimentaires.
